# EUROPEAN PATENT SPECIFICATION

(11) **EP 2 459 635 B1**
(45) Date of publication and mention of the grant of the patent: **08.05.2013**
(21) Application number: 10709006.0
(22) Date of filing: 17.03.2010
(51) Int. Cl.: C08K 5/00, C08K 5/05, C08K 5/098, C08L 33/14, C08J 3/12, A61K 9/28

(54) **POWDERY OR GRANULATED COMPOSITION COMPRISING A COPOLYMER, A SALT OF A FATTY MONOCARBOXYLIC ACID AND A FATTY MONOCARBOXYLIC ACID AND/OR A FATTY ALCOHOL**
PULVRIGE ODER GRANULÖSE ZUSAMMENSETZUNG MIT EINEM COPOLYMER, EINEM SALZ EINER MONOCARBONFETTSÄURE UND EINER MONOCARBONFETTSÄURE UND/ODER EINEM FETTALKOHOL
COMPOSITION SOUS FORME DE POUDRE OU DE GRANULÉS COMPRENANT UN COPOLYMÈRE, UN SEL D'UN ACIDE GRAS MONOCARBOXYLIQUE ET UN ACIDE GRAS MONOCARBOXYLIQUE ET/OU UN ALCOOL GRAS

(30) Priority: 30.07.2009 WO PCT/EP2009/059861
(43) Date of publication of application: 06.06.2012
(73) Proprietor: Evonik Röhm GmbH, 64293 Darmstadt (DE)
(72) Inventor: ROTH, Erna, 64297 Darmstadt (DE); ALEXOWSKY, Rüdiger, 64569 Nauheim (DE); PETEREIT, Hans-Ulrich, 64291 Darmstadt (DE); MEIER, Christian, 64295 Darmstadt (DE)
(86) International application number: PCT/EP2010/053447
(87) International publication number: WO 2011/012335

(56) References cited:
- US-A1- 2003 064 036
- US-A1- 2005 281 871
- US-B1- 6 576 255

## Description

### Field of the invention

The present invention is concerned with a powdery or granulated composition comprising a copolymer, a salt of a fatty monocarboxylic acid and/or a fatty monocarboxylic acid and/or a fatty alcohol as ready to use aqueous dispersion for the coating or binding of active ingredients in the field of pharmacy, nutraceuticals or cosmetics.

### Technical background

WO02067906A1 (US20030064036A1) describes a coating and binding agent with improved storage stability, consisting essentially of
(a) a copolymer, consisting of radically polymerized C₁- to C₄-alkyl esters of acrylic or methacrylic acid and other alkyl(meth)acrylate monomers which comprise functional tertiary amino groups, the copolymer being in the form of a powder with an average particle size of 1 -40 µm,
(b) 3-15 wt.%, based on (a), of an emulsifier with a HLB value of at least 14,
(c) 5-50 wt.-%, based on (a), of a C12-C18-monocarboxylic acid or a C12-C18-hydroxyl compound.

One of the beneficial effects of the invention is that the vapour permeability is reduced. Compound (a) is preferably EUDRAGIT® EPO. A preferred compound (b) in the examples is Sodium-Laurylsulfate, which can be used together with lauric acid, stearic acid or lauryl alcohol as compound (c). Dispersion processing times of the inventive examples are around 3 to 6 hours.

### Problem and Solution

There is a permanent need for improved coating and binding agents for pharmaceutically, nutraceutically or cosmetically purposes. Customers prefer ready to use powdery or granulated compositions comprising suitable copolymers which can be used for coating or binding processes after dispersing them in water.

General problems are that additives like emulsifiers must be added to the copolymers to be used for coating or binding processes in order to allow a rapid dispersion times. However additives which allow rapid dispersion times may on the other hand sometimes effect the viscosity of the dispersion in negative way or increase the water vapor permeability. Especially if the viscosity of the dispersion is too high this may lead to problems in the subsequently coating or binding process.

Furthermore some frequently used additives like for instance sodium laurylsulfate (s. WO02067906A1) although in general suitable and accepted for pharmaceutical purposes, are in the meantime regarded as showing a too high level of toxicity. This may depend on the total amount of the polymer and additive composition that is present in a daily dosage of the intended pharmaceutical, nutraceutical or cosmetical form. However in general additives with a toxicity as low as possible are of course preferred.

Thus it is one object of the present invention to provide powdery or granulated compositions for coating or binding purposes that get completely dispersed in water with a processing time as short as possible. The additives employed to support the rapid dispersion time shall show a toxicity level as low as possible. Furthermore the viscosity of the dispersion must be in a range which allows subsequently successful coating or binding procedures.

The problem is solved by a powdery or granulated composition comprising at least by 30 % by weight of a mixture of
(a) a copolymer composed of polymerized units of C₁- to C₄-alkyl esters of acrylic or methacrylic acid and of alkyl(meth)acrylate monomers with a tertiary amino group in the alkyl radical and
(b) 5 to 28 % by weight based on (a) of a salt of a fatty monocarboxylic acid having 10 to 18 carbon atoms, and
(c) 10 to 30 % by weight based on (a) of fatty monocarboxylic acid having 8 to 18 carbon atoms and/or a fatty alcohol having 8 to 18 carbon atoms.

The inventive composition is intended to be used as a rapidly in water dissolving powder or granulate. The dispersed aqueous compositions show a low viscosity and can therefore be processed directly as coating and binding agents for pharmaceutically, nutraceutically or cosmetically purposes. Preferred embodiments can be prepared as dispersions with dry weight contents of up to 30 % (weight/volume). The main components (a), (b) and (c) preferably show extremely low toxicity data in the range 2000 mg/kg LD50 (rat) or even less toxic.

### Component (a)

Component (a) is a copolymer composed of polymerized units of C₁- to C₄-alkyl esters of acrylic or methacrylic acid and of alkyl(meth)acrylate monomers with a tertiary amino group in the alkyl radical.

### Amino Methacrylat Copolymer

The copolymer component (a) may be a so called "amino methacrylate copolymer (USP/NF)", "basic butylated methacrylate copolymer (Ph. Eur)" or "aminoalkyl Methacrylate Copolymer E (JPE)" which are of the EUDRAGIT® E type. Suitable EUDRAGIT® E type copolymers are known, for example, from EP 0 058 765B1.

The amino (meth)acrylate copolymer may be composed, for example, of 30 to 80% by weight of free-radically polymerized C₁- to C₄-alkyl esters of acrylic acid or of methacrylic acid, and 70 to 20% by weight of (meth)acrylate monomers having a tertiary amino group in the alkyl radical.

Suitable monomers with functional tertiary amino groups are detailed in US 4 705 695, column 3 line 64 to column 4 line 13. Mention should be made in particular of dimethylaminoethyl acrylate, 2-dimethylaminopropyl acrylate, dimethylaminopropyl methacrylate, dimethylaminobenzyl acrylate, dimethylaminobenzyl methacrylate, (3-dimethylamino-2,2-dimethyl)propyl acrylate, dimethylamino-2,2-dimethyl)propyl methacrylate, (3-diethylamino-2,2-dimethyl)propyl acrylate, diethylamino-2,2-dimethyl)propyl methacrylate and diethylaminoethyl methacrylate.
Particular preference is given to dimethylaminoethyl methacrylate.

The content of the monomers with tertiary amino groups in the copolymer may advantageously be between 20 and 70% by weight, preferably between 40 and 60% by weight. The proportion of the C₁- to C₄-alkyl esters of acrylic acid or methacrylic acid is 70 - 30% by weight. Mention should be made of methyl methacrylate, ethyl methacrylate, butyl methacrylate, methyl acrylate, ethyl acrylate and butyl acrylate.

A suitable amino (meth)acrylate copolymer may be polymerized out of, for example, from 20 - 30% by weight of methyl methacrylate, 20 - 30% by weight of butyl methacrylate and 60 - 40% by weight of dimethylaminoethyl methacrylate.

A specifically suitable commercial amino (meth)acrylate copolymer is, for example, formed from 25% by weight of methyl methacrylate, 25% by weight of butyl methacrylate and 50% by weight of dimethylaminoethyl methacrylate (EUDRAGIT® E100 or EUDRAGIT® E PO (powder form)). EUDRAGIT® E100 and EUDRAGIT® E PO are water-soluble below approx. pH 5.0 and are thus also gastric juice-soluble.

### Component (b)

Component (b) is a, one or more, salt of a fatty monocarboxylic acid having 10 to 18 carbon atoms. Suitable amounts are 5 to 28, preferably 5 to 25, preferably 5 to 20, preferably 5 to15 or preferred 8 to 12 % by weight based on the copolymer component (a). As a rule the salt of a fatty monocarboxylic acid having 10 to 18 carbon atoms is water soluble or water dispersible.

In relation to the cationic groups in the polymer component (a) the component (b) may be present in a molar ratio of 5 to 35, preferably 5 to 25 or preferably 12-25 mol-%.

In a further preferred embodiment of the present invention the salt in respect to component (b) is selected from the group consisting of alkali metal salt or an ammonium salt.

In a particularly preferred embodiment of the present invention, the salt in respect to component (b) is a salt of a saturated, preferably unbranched, preferably unsubsituted, mono carboxylic acid (fatty acid) having 10 to 18, preferably 10 to 14 or 16 to 18 carbon atoms which may be selected from the group of consisting of the salts of capric acid, lauric acid, myristic acid, palmitic acid, or stearic acid or mixtures thereof. Even more preferred is an alkali metal salt or ammonium salt thereof. Even further preferred is a salt of capric acid, particularly preferred is sodium capric acid = sodium caprate (C₉H₁₉COO⁻Na⁺),

The salts of the following saturated monocarbonic acids are suitable for the purposes of the invention:
C₁₀: capric acid (C₉H₁₉COOH),
C₁₂: lauric acid (C₁₁H₂₃COOH),
C₁₄: myristic acid (C₁₃H₂₇COOH),
C₁₆: palmitic acid (C₁₅H₃₁COOH),
C₁₈: stearic acid (C₁₇H₃₅COOH)

Salts of organic or anorganic acids other than salts of mono carboxylic acids (fatty acids) having 10 to 18 carbon atoms are assumed to be not suitable for the purposes the present invention.

Saturated, mono carboxylic acids (fatty acids) having 10 to 18 carbon atoms are not suitable for the purposes of the invention as long as they are not applied together with an alkali metal or an ammonium hydroxide to react in situ to the salt form.

The salt of a saturated, preferably unbranched, mono carboxylic acid (fatty acid) having 10 to 18, preferably 10 to 14 or 16 to 18 carbon atoms is preferably unsubsituted.

It is understood that all the salts of a saturated, preferably unbranched, preferably unsubsituted, mono carboxylic acid (fatty acid) having 10 to 18, preferably 10 to 14 or 16 to 18 carbon atoms which are suitable in the sense of the present invention should be acceptable as a pharmaceutical or nutraceutical ingredient.
A salt of a fatty monocarboxylic acid having 10 to 18 carbon atoms may also be generated by adding the corresponding acid as component (c) and base, for instance sodium hydroxid (NaOH) or potassium hydroxid (KOH). This results in a balance between the acid of the fatty monocarboxylic acid (component (c)) and the corresponding salt (component (b)) by in situ salt formation. The amount of base needed can be determined by calculation of the degree of molar neutralisation.

### Component (c)

Component (c) is a, one or more, fatty monocarboxylic acid having 8 to 18 carbon atoms and/or a, one or more, fatty alcohol having 8 to 18 carbon atoms. Suitable amounts are 10 to 30, preferably 10 to 28, preferably 10 to 20 or preferred 12 to 18 % by weight based on the copolymer component (a).

In relation to the cationic groups in the polymer component (a) the component (c) may be present in a molar ratio of 10 to 50, preferably 15-40 mol-%.

### Fatty monocarboxylic acid having 8 to 18 carbon atoms

the following monocarbonic acids are suitable for the purposes of the invention:
C₈: caprylic acid (C₇H₁₅COOH),
C₁₀: capric acid (C₉H₁₉COOH)
C₁₂: lauric acid (C₁₁H₂₃COOH)
C₁₄: myristic acid (C₁₃H₂₇COOH)
C₁₆: palmitic acid (C₁₅H₃₁COOH),
C₁₈: stearic acid (C₁₇H₃₅COOH) /

Saturated, preferably unbranched, mono carboxylic acid (fatty acid) having 8 to 18, preferably 8 or 10 or 16 or 18 carbon atoms are preferably unsubsituted. Preferred are capric acid (C₉H₁₉COOH) or stearic acid (C₁₇H₃₅COOH) as single components (c) or mixtures thereof, most preferred in combination with sodium caprate (C₉H₁₉COONa) as component (b)

### Fatty alcohol having 8 to 18 carbon atoms

The following C₈ - C₁₈ fatty alcohols are suitable for the purposes of the invention:
C₈: capryl alcohol (1-octanol)
C₈: 2-ethyl hexanol (branched)
C₉: pelargonic alcohol (1-nonanol)
C₁₀: capric alcohol (1-decanol, decyl alcohol)
C₁₁: undecanol
C₁₂: lauryl alcohol (1-dodecanol)
C₁₄: myristyl alcohol (1-tetradecanol)
C₁₆: cetyl alcohol (1-hexadecanol)
C₁₆: palmitoleyl alcohol (cis-9-hexadecen-1-ol; unsaturated)
C₁₈: stearyl alcohol (1-octodecanol)
C₁₈: isostearyl alcohol (16-methylheptadecan-1-ol; branched)
C₁₈: elaidyl alcohol (9E-octadecen-1-ol; unsaturated)
C₁₈: oleyl alcohol (cis-9-octadecen-1-ol; unsaturated)
C₁₈: linoleyl alcohol (9Z, 12Z-octadecen-1-ol; polyunsaturated)
C₁₈: elaidolinoleyl alcohol (9E, 12E-octadecadien-1-ol; polyunsaturated)
C₁₈: linolenyl alcohol (9Z, 12Z, 15Z-octadecatrien-1-ol; polyunsaturated)
C₁₈: elaidolinolenyl alcohol (9E, 12E, 15-E--octadecatrien-1-ol; polyunsaturated)
C₁₈: ricinoleyl alcohol (12-hydroxy-9-octadecen-1-ol; unsaturated, diol)

Preferred are C₈- C₁₀ fatty alcohols. Most preferred is capryl alcohol (1-octanol) and dodecanol.

### Pharmaceutical, nutraceutical or cosmetical excipients

The compositions according to the invention are further characterised in that up to 200 %, up to 70 %, up to 60 %, up to 50 %, up to 40 %, up to 30 %, up to 20 % or up to 10 % by weight based on the total weight of the components (a), (b) and (c) of pharmaceutical, nutraceutical or cosmetical excipients which are different from the components (a), (b) and (c) may be contained. However the composition according to the invention may as well contain any or essentially any pharmaceutical, nutraceutical or cosmetical excipients. Thus the composition may essentially consist or consist to 100 % of the components (a), (b) and (c).

The term pharmaceutical, nutraceutical or cosmetical excipients is well known to the skilled person. Such excipients are customary in pharmacy but also in the field of nutraceuticals or cosmetics, occasionally also they are referred as customary additives. It is, of course, always necessary for all the excipients or customary additives employed to be toxicologically acceptable and usable in particular in food or in medicaments without a risk for customers or patients.

Although the requirements are usually higher in the pharmaceutical field there is a widely overlap of excipients used for pharmaceutical purposes and those used for nutraceutical purposes. Usually all pharmaceutical excipients may be used for **nutraceutical** purposes and at least a large number of nutraceutical excipients are allowed to be used for pharmaceutical purposes as well. Excipients may be are added to the formulation of the invention, preferably during the mixing of the powders production of the granules, for the coating or binding of active ingredients, coating of solids or patches or dispersing semi solids.

Pharmaceutical, **nutraceutical** or cosmetical excipients with are different from the components (a), (b) and (c) may be contained for practical reasons, for instance to avoid stickiness or to add a colour. However these excipients usually do not contribute or do show any or almost no effect on the invention itself as claimed here.

Pharmaceutical, nutraceutical or cosmetical excipients with are different from the components (a), (b) and (c) do not contribute to the invention in a narrow sense which is based on the interaction of the components (a), (b) and (c). Pharmaceutical, nutraceutical or cosmetical excipients with are different from the components (a), (b) and (c) and which may have an essential adverse effect on the major beneficial effects of the present invention e.g. the preparation time or on the viscosity of the dispersion should be avoided and can be excluded. For instance the addition of essential amounts sodium dodecylsulfate or similar substances with emulgator properties different from the components (b) and (c) should be avoided. Preferably any addition of sodium dodecylsulfate or similar substances with emulgator properties different from the components (b) and (c) should be avoided.

Typical pharmaceutical, nutraceutical or cosmetical excipients with are different from the components (a), (b) and (c) are familiar to those skilled in the art. Examples are antioxidants, brighteners, flavouring agents, flow aids, fragrances, glidants (release agents), penetration-promoting agents, pigments, plasticizers, polymers, pore-forming agents or stabilizers. They may be used as processing adjuvants and are intended to ensure a reliable and reproducible preparation process as well as good long-term storage stability, or they achieve additional advantageous properties in the pharmaceutical form. They are added to the polymer formulations before processing and can influence the permeability of the coatings. This property can be used if necessary as an additional control parameter.

Anionic polymers or anionic (meth)acrylate copolymers which could interact with the polymer component (a) may be excluded. Dicarboxylic acids having 3 to 10 carbon atoms may be excluded as well.

### Plasticizers

Plasticizers achieve through physical interaction with a polymer a reduction in the glass transition temperature and promote film formation, depending on the added amount. Suitable substances usually have a molecular weight of between 100 and 20 000 and comprise one or more hydrophilic groups in the molecule, e.g. hydroxyl, ester or amino groups.

Examples of suitable plasticizers are alkyl citrates, glycerol esters, alkyl phthalates, alkyl sebacates, sucrose esters, sorbitan esters, diethyl sebacate, dibutyl sebacate and polyethylene glycols 200 to 12 000. Preferred plasticizers are triethyl citrate (TEC), acetyl triethyl citrate (ATEC), diethyl sebacate and dibutyl sebacate (DBS). Mention should additionally be made of esters which are usually liquid at room temperature, such as citrates, phthalates, sebacates or castor oil. Esters of citric acid and sebacinic acid are preferably used.

Addition of the plasticizers to the formulation can be carried out in a known manner, directly, in aqueous solution or after thermal pre-treatment of the mixture. It is also possible to employ mixtures of plasticizers.

### Glidants / Release Agents / Detackifier:

Glidants, release agents or detackifiers usually have lipophilic properties and are usually added to spray suspensions. They prevent agglomeration of cores during film formation. There are preferably used talc, Mg or Ca stearate, ground silica, kaolin or nonionic emulsifiers with an HLB value of between 2 and 8. Standard proportions for use of release agents in the inventive coating and binding agents range between 0.5 and 70 wt % relative to the components (a), (b) and (c).

### Pigments:

Only rarely is the pigment added in soluble form. As a rule, aluminium oxide or iron oxide pigments are used in dispersed form. Titanium dioxide is used as a whitening pigment. Standard proportions for use of pigments in the inventive coating and binding agents range between 20 and 200 wt % relative to the components (a), (b) and (c).

Of course all kind of excipients used must of course be toxicologically safe and to be used in nutraceuticals or pharmaceuticals without risk for customers or patients.

### The Preparation Process

Process for preparing a composition according to the invention may be characterized in that the components (a), (b) and (c) are intermixed with each other by powder mixture, dry granulation, wet granulation, melt granulation, spray drying or freeze drying.

Components (a), (b) and (c) may be intermixed with each other in a powdery stage or by a granulations process, which can be a dry, a wet or melt granulation process. As an alternative, the components can be added subsequently in the aqueous dispersing phase

### Powder mixture process

Components (a), (b) and (c) are intermixed with each other in a powdery stage by using mixer equipment. Powdery stage can be defined in that the particle of components may have an average particle size of less than 1 mm, preferably of less than 0.5 mm, especially of 100 µm or less, preferably in the range 10 to 100 µm. The process of powder mixing is well known to a skilled person. The average particle size may be determined by sieving techniques or by laser diffraction methods.

### Dry granulation process

Components (a), (b) and (c) are intermixed with each other in a form of granulates by using a mixer equipment. Granulates may have an average particle size of 1 mm or more, preferably in the range of 1 to 5 mm.

### Wet granulation process

Powders or granules of components (a), (b) and (c) are intermixed with each other in a wet stage by wetting the powders or granulates with water or organic solvents and then using a mixer or kneading equipment. Wet stage shall mean that there is a wet mass than can be manually kneaded with a water content for instance in the range 10 to 100 % by weight. After wetting and mixing respectively kneading the wet mass is dried and then again commuted to granules or powders. The process of wet granulation is well known to a skilled person. Solutions of the components (a), (b) or (c) or combinations thereof in organic solvents like methanol, ethanol, isopropanol, ethyl acetate or acetone may also be used in the wet granulation process. The organic solvents may optionally contain up to 50 % (v/v) of water.

### Melt granulation process

Powders or granules of components (a), (b) and (c) are intermixed with each other usually without the addition of solvents at elevated temperatures where at least the copolymer is in a molten stage. This can be performed in a heated mixer or in an extruder, preferably in a twin screw extruder. After mixing the molten mass is cooled and then again commuted to granules or to powders. The process of melt granulation is well known to a skilled person.

### Spray drying or freeze drying process

Components (a), (b) and (c) are dissolved or dispersed in water or in organic solvents or mixtures of water with organic solvents, separately or as premix and subsequently dried and probably sieved. The compounds may have an average particle size in the range of 10 µm to 2 mm or more, preferably in the range of 20 µm to 1.5 mm.

### Dispersion or solution process

The components (a), (b) and (c) are added to the aqueous dispersing or solution agent, preferably purified water, as powder mixtures, granules or single one after another while gentle stirring with a conventional stirrer at room temperature. Advantageously, according to this invention, the need of a high shear mixer or specific disperser will not be necessary. Additionally, the heating of the suspension will be not necessary. After stirring of less than 3 hours dispersions or solutions are formed being able to be sprayed in coating or granulation processes and/or to form films after drying. The dispersion or solution may have a total content of solids less than 35% by weight, preferably less than 25% by weight and pH -values between 7 and 11. The pH values of the dispersion or solution may in the range from 8 to 10, preferably from 9 to 10.

### Dispersion preparation time

The dispersion preparation time can for instance be observed and determined by polarization microscopy. The time when the dry powdery or granulate mixture is stirred into the water is defined as starting point. The dispersing aqueous mixture is further stirred at room temperature (ca. 22°C). At the beginning there is a turbid dispersion, that becomes first white and then more and more clear during stirring. Drops of the dispersing aqueous mixture are then taken every 10 minutes and observed under a polarization microscope with a magnification of 100-fold with the support of a phase filter. The time point when no or almost no particles (at least less than ten particles in the view field) are observed in the fluid of such a drop under the microscope is taken as end point of the dispersion process. The accuracy of this determination method is in most cases sufficient to differ the preparation times of the different dispersion preparations apart from each other. The inventive composition may be characterized by a dispersion or solution preparation time of less than 3 hours, preferably 2.5 hours or less most preferred 1.5 hours or less. The preparation time is starting from adding the dry powdery or granulate mixture into water at room temperature, further stirring and thereby dissolving the components to end up at a clear solution or dispersion respectively.

### Practical Applications:

Dispersions according to this invention may be used in granulation or coating process in the development and manufacturing of nutrition supplements, nutraceuticals, cosmetics, cosmeceuticals, pharmaceutical intermediates or pharmaceuticals. Due to the physicochemical properties of the polymer, which are maintained in the dispersed compounds of this invention, functions such as coloring, taste masking, moisture protection, light protection, odor masking or eased swelling are introduced into the final dosage form.

Application procedures and processes known to the skilled person and published for example in:
G. Cole, J. Hogan, M. Aulton, Pharmaceutical coating Technology Taylor & Francis, 1995
K. H. Bauer, K. Lehmann, H. P. Osterwald, G. Rothgang, "Coated Dosage Forms", CRC Press 1998
Pharmaceutical Manufacturing Encyclopedia, William Andrew Publishing; Third Edition, 2005
Encyclopedia of Pharmaceutical Technology, Third Edition, Informa Healthcare, 2006
J. W. McGinity, L. A. Felton, aqueous Polymeric Coatings for Pharmaceutical Dosage Forms, Third Edition, Informa Healthcare, 2008

### Nutraceuticals

Nutraceuticals can be defined as extracts of foods claimed to have medical effects on human health. The **nutraceutical** is usual contained in a medical format such as capsule, tablet or powder in a prescribed dose. Examples for nutraceuticals are resveratrol from grape products as an antioxidant, soluble dietary fiber products, such as psyllium seed husk for reducing hypercholesterolemia, broccoli (sulphane) as a cancer preservative, and soy or clover (isoflavonoids) to improve arterial health. Other nutraceuticals examples are flavonoids, antioxidants, alpha-linoleic acid from flax seed, beta-carotene from marigold petals or antocyanins from berries. Sometimes the expression neutraceuticals is used as synonym for nutraceuticals.

### Cosmetics

Cosmetics are substances used to enhance or protect the appearance or odor of the human body. Typical cosmetical active ingredients may comprise vitamins, phytochemicals, enzymes, antioxidants, and essential oils. Cosmetics may include skin-care creams, lotions, powders, perfumes, lipsticks, fingernail and toe nail polish, eye and facial makeup, permanent waves, colored contact lenses, hair colors, hair sprays and gels, deodorants, baby products, bath oils, bubble baths, bath salts, butters and many other types of products. Their use is widespread, especially among women but also by men. A subset of cosmetics is called "make-up," which refers primarily to colored products intended to alter the user's appearance. Many manufacturers distinguish between decorative cosmetics and care cosmetics. The term cosmetics shall include topically applied forms such as so called cosmeceuticals as well as orally ingested forms such as so called nutricosmetics.

### Active ingredients

The inventive composition may be used as a coating and binding agent in combination with all kinds of pharmaceutical, **nutraceutical** or cosmeceutical active ingredients. However additionally beneficial effects may be gained in combination with those kinds of active ingredients, which need to be formulated in a taste masked form or in a moisture resistant form.

Pharmaceutically, nutraceutically or cosmetically active ingredients have in common that they are active ingredients which have a positive effect on the health of an organism, e.g the human health. They have also in common that their formulations are often the same or very similar. Often also the same kind of excipients or additives are used in combination with these kind of active ingredients. Pharmaceutically active ingredients are used to cure diseases and effect the health of an organism, e.g the human health more or less directly. Nutraceutical active ingredients are used to supplement the nutrition and thus support the health of an organism, e.g the human or animal health indirectly. Cosmetically active ingredients are meant to support the human health indirectly for instance by balancing the water content of the human skin.

### Process

The invention also relates to a process for preparing the inventive composition, characterized in that the components (a), (b) and (c) are intermixed with each other by powder mixture, dry granulation, wet granulation or melt granulation. In the case of wet granulation and the components (a), (b) or (c) or combinations thereof may be used in the form an organic solution.

### Use

The invention discloses the use of the composition as a coating or binding agent for the spray coating or binding of pharmaceutical, nutraceutical or cosmetical compositions. Preferred active ingredient containing compositions may be in the form of powders, pellets, granules, minitablets, sachets, dry syrups, tablets or capsules or nutraceutical compositions or cosmetical compositions. The use as a coating solution shall include the use as a subcoat or a topcoat in combination with other coatings.

### Examples

The following copolymers were used in the Examples.

### Copolymer :

BASIC BUTYLATED METHACRYLATED COPOLYMER EUDRAGIT^{®} E PO or EUDRAGIT^{®} E 100.

EUDRAGIT^{®} E is a copolymer composed of 25% by weight of methyl methacrylate, 25% by weight of butyl methacrylate and 50% by weight of dimethylaminoethyl methacrylate.

### Model drug

Studies were conducted using tablets (300mg) with quinidine sulphate (immediate bitter taste) or silicagel (550 mg total weight, 11 mm diameter) as marker..

### Excipients

All excipients were used in pharmaceutical quality

### Disintegration studies:

Disintegration was tested according USP 28 <701> Disintegration

### Dissolution studies

Coated tablets were tested according to
USP 28-NF23, General Chapter <711>, *Dissolution,*

### Dissolution parameters:

Apparatus: USP Type-II (Paddle)
RPM: 50/min.
Temperature: 37.5 ± 0.5 ° C
Dissolution volume: 900 ml.
Wavelength: 250 nm

### Dissolution medium 1:

0.1 molar Hydrochloric acid (HCl), (European Pharmacopoeia = EP)

### Dissolution medium 2:

Phosphate buffer pH 6.0 (European Pharmacopoeia = EP)

### Results

The following tables explain formulation examples 1 - 25 according to the invention as well as non inventive comparative examples:
Dispersion are prepared by adding the component (b), (a) and (c) in this order separately or as a granulated or blended premixture of all components in purified water in a quantity, providing the specified dry solid content. Stirring was performed with a magnetic stirrer or a simple agitator providing low shear forces.

In examples 23, 24 and 25 organic solvents are used for granulation. EUDRAGIT^{®} E 100 was dissolved in isopropanol (95% w/w) to form a 15% (w/w) solution while gentle stirring. The components (b) and (c) were added subsequently and stirred until complete dissolution. In case a glidant was used too, it was added to the clear solution and shortly stirred to get a homogeneous suspension. The final suspension was dried completely in a vacuum oven at 50°C for 24 h. The dried film was milled to get a powder of ca 0.5 mm particle diameter. The powder was tested accordingly to examples 1 to 22.

**Table 1**

| Components | Example | 1 | 2 | 3 | 4 | 5 | 6 | 7 | 8 | 9 | 10 | 11 |
|---|---|---|---|---|---|---|---|---|---|---|---|---|
| Component a.) | EUDRAGIT® E PO | 100 | 100 | 100 | 100 | 100 | 100 | 100 | 100 | 100 | 100 | 100 |
| Component b.) calculated on a.) weight [%] | Sodium caprate (C₁₀) | 10 (16.0) | 15 (24.1) | 15 (24.1) | 12 (19.3) | - | 15* (24.1) | 10 (16.0) | 15 (24.1) | 5 (8.0) | 15 (24.1) | 10 (16.0) |
| (mol [%]) | Sodium stearate (C₁₈) | - | - | - | - | 15 (15.3) | - | - | - | - | - | - |
| Component c.) calculated on a.) weight [%] | Stearic acid (C₁₈) | 15 (16.4) | 15 (16.4) | - | - | 15 (16.4) | 15 (16.4) | - | - | - | - | - |
| (mol [%]) | Capric acid (C₁₀) | - | - | 20 (36.2) | 10 (18.1) | - | - | - | - | - | - | - |
| | 1-Octanol (C₈) | - | - | - | - | - | - | 15 (35.9) | 20 (47.9) | 20 (47.9) | - | - |
| | 1-Dodecanol (C₁₂) | - | - | - | - | - | - | - | - | - | 15 (25.1) | 15 (25.1) |
| Content (a+b+c) [%] | | 100 | 100 | 100 | 100 | 100 | 100 | 100 | 100 | 100 | 100 | 100 |
| Content of other exipients [%] | | - | - | - | - | - | - | - | - | - | - | - |
| Preparation time [h] | | 1.8 | 1.5 | 2.0 | 1.5 | 2.5 | 2.0 | 0.5 | 0,5 | 2.0 | 2.5 | 1.0 |
| Dry content in dispersion weight [%] | 15 | x | x | - | x | x | x | - | - | - | - | - |
| | 20 | - | - | x | - | - | - | - | - | - | x | - |
| | 30 | - | - | - | - | - | - | x | x | x | - | x |
| Viscosity | | x | x | xx | x | xx | x | x | x | x | | x |
| low = x; middle = xx; high = xxx | | | | | | | | | | | | |

| | | | | | | | | | | | | |
|---|---|---|---|---|---|---|---|---|---|---|---|---|
| * In situ preparation of sodium caprate by dispersing 12.2 g capnc acid in water and adding 70.7 ml 1M NaOH (mol [%])=(mol [%]) of components (b) or (c) in relation to the cationic groups in the polymer component (a). | | | | | | | | | | | | |

**Table 2**

| Components | Example | 12* | 13 | C14 | C15 | C16 | C17 |
|---|---|---|---|---|---|---|---|
| Component a.) | EUDRAGIT® E PO | 100 | 100 | 100 | 100 | 100 | 100 |
| Component b.) calculated on a.) weight [%] | Sodium caprate(C₁₀) | 12 (19.3) | 10 (16.0) | 12 (19.3) | - | - | 4 (6.4) |
| (mol [%]) | | | | | | | |
| Component c.) calculated on a.) weight [%] | Stearic acid (C₁₈) | - | 15 (16.4) | - | 15 (16.4) | 15 (16.4) | - |
| (mol [%]) | Capric acid (C₁₀) | 10 (18.1) | - | 5 (9.0) | - | - | - |
| | 1-Octanol (C₈) | - | - | - | - | - | 15 (35.9) |
| Content (a+b+c) [%] Non-inventive emulgator | (a+b+c) | 100 | 75.75 | 100 | - | - | 100 |
| | Sodium lauryl sulfate | - | - | - | 10 (10.8) | 10 (10.8) | - |
| Content of other exipients [%] | | - | 24.25 | - | - | - | - |
| Other exipients calculated on a.) weight [%] | Talc | - | 30 | - | - | - | - |
| | Candurin red lustre | - | 10 | - | - | - | - |
| Preparation time [h] | | 1.3 | 2.0 | >4 | >4 | >4 | >4 |
| Dry content in dispersion weight [%] | 15 | - | x | x | x | - | - |
| | 20 | x | - | - | - | - | - |
| | 30 | | - | - | - | x | x |
| Viscosity | | x | x | - | x | xxx | - |
| low = x; middle = xx; high = xxx | | | | | | | |
| Observation | | - | - | no dispersion to low c.) | No dispersion | No dispersion gel | no dispersion to low b.) |

| | | | | | | | |
|---|---|---|---|---|---|---|---|
| * Extruded compound of formulation from example 4 with EUDRAGIT^{®} E 100 (mol [%])=(mol [%]) of components (b) or (c) in relation to the cationic groups in the polymer component (a). | | | | | | | |

**Table 3**

| Components | Example | C18 | C19 | C20 | C21 | C22 |
|---|---|---|---|---|---|---|
| Component a) | EUDRAGIT® E PO | 100 | 100 | 100 | 100 | 100 |
| Non-inventive salt of fatty acid calculated on a) weight [%] | Sodium arachidate(C₂₀) | 10 (9.3) | - | - | - | - |
| (mol [%]) | Sodium caprylate(C₈) | - | 10 (18.8) | - | - | 10 (18.8) |
| | Disodium succinate (C₄) | - | - | 10 (19.2) | - | - |
| Component b) fatty acid salt calculated on a) weight [%] (mol [%]) | Sodium caprate(C₁₀) | - | - | - | 10 (16.0) | - |
| Non-inventive fatty acid calculated on a.) weight [%] | Arachidic acid (C₂₀) | - | - | - | 15 (15.0) | - |
| (mol [%]) | Hexanoic acid (C₆) | - | - | - | - | 15 (40.3) |
| Component c.) fatty acid/ fatty alcohol calculated on a.) weight [%] | Stearic acid (C₁₈) | 17 (18.6) | 15 (16.4) | 15 (16.4) | | |
| (mol [%]) | | | | | - | - |
| Content [%] | (a+b+c) | 100 | 100 | 100 | 100 | 100 |
| Content of other exipients [%] | | - | - | - | - | - |
| Preparation time [h] | | >4 | >4 | >4 | >4 | >4 |
| Dry content in dispersion weight [%] | 15 | x | x | x | x | x |
| | 20 | - | - | - | - | - |
| | 30 | - | - | - | - | - |
| Viscosity | | | | | | |
| low = x; middle = xx; high = xxx | | xxx | xxx | xxx | xxx | |
| Observation | | no dispersion /solution | no dispersion /solution | no dispersion /solution | no dispersion /solution | no dispersion /solution |

| | | | | | | |
|---|---|---|---|---|---|---|
| (mol [%])=(mol [%]) of components (b) or (c) in relation to the cationic groups in the polymer component (a). | | | | | | |

**Table 4**

| Components | Example | 23 | 24 | 25 |
|---|---|---|---|---|
| Component a.) | EUDRAGIT® E PO | 100 | 100 | 100 |
| Component b.) calculated on a.) weight [%] | Sodium caprate (C₁₀) | 10 (16.0) | 12 (19.3) | 12 (19.3) |
| (mol [%]) | | | | |
| Component c.) calculated on a.) weight [%] | Capric acid (C₁₀) | - | 10 (18.1) | 10 (18.1) |
| (mol [%]) | 1-Dodecanol (C₁₂) | 15 (25.1) | - | - |
| Content (a+b+c) [%] | | 100 | 100 | 100 |
| Content of other excipients calculated on a.) weight [%] | Syloid 244 FP | - | 35 | - |
| | Magnesium stearate | 35 | - | - |
| Isopropanol [%] | | - | | |
| Demineralized water [%] | | - | | |
| Preparation time [h] | | 1.0 | 1.3 | 1.3 |
| Dry content in dispersion weight [%] | 15 | - | - | - |
| | 20 | x | x | x |
| | 30 | - | - | - |
| Viscosity | | x | x | x |
| low = x; middle = xx; high = xxx | | | | |
| Observation | | dispersion | dispersion | solution |

| | | | | |
|---|---|---|---|---|
| (mol [%])=(mol [%]) of components (b) or (c) in relation to the cationic groups in the polymer component (a). | | | | |

**Table 5**

| Components | Example | 26 | 27 | 28 | 29 | 30 | 31 | 32 | 33 | C34 |
|---|---|---|---|---|---|---|---|---|---|---|
| Component a.) | EUDRAGIT® E PO | 100 | 100 | 100 | 100 | 100 | 100 | 100 | 100 | 100 |
| Component b.) calculated on a.) weight [%] | Sodium caprate (C₁₀) | 10 (16.0) | 15 (24.1) | | 20 (32) | 20 (32) | 10* (16) | - | 15 (24.1) | 15 (24.1) |
| (mol [%]) | Sodium laurate (C₁₂) | | | | | | | 17 (23.9) | - | - |
| | Sodium stearate (C₁₈) | 8 (8.1) | 4 (4.1) | 17 (17.3) | - | - | 10 (10.2) | - | 10 (10.2) | 15 (15.3) |
| Component c.) calculated on a.) weight [%] | Stearic acid (C₁₈) | 10 (10.9) | 15 (16.4) | 15 (16.4) | - | 15 (16.4) | 10 (10.9) | - | 15 (16.4) | 15 (16.4) |
| (mol [%]) | Capric acid (C₁₀) | 10 (18.1) | - | | 15 (27.1) | - | - | - | - | - |
| | 1-Octanol (C₈) | - | 5 (12) | - | - | - | 10 (23.9) | 15 (35.9) | - | - |
| | 1-Dodecanol (C₁₂) | - | - | 10 (19.7) | - | - | - | - | - | - |
| Content (a+b+c) [%] | | 100 | 100 | 100 | 100 | 100 | 100 | 100 | 100 | 100 |
| Content of other exipients [%] | | - | - | - | - | - | - | - | - | - |
| Preparation time [h] | | 1.8 | 1.5 | 4.0 | 1.5 | 1.5 | 2.0 | 1.5 | 2.5 | 2.8 |
| Dry content in dispersion weight [%] | 15 | x | x | x | | x | x | - | x | x |
| | 20 | - | - | - | x | - | - | x | - | - |
| | 30 | - | - | - | - | - | - | - | - | - |
| Viscosity | | x | x | xx | x | xx | x | x | xx | xxx |
| low = x; middle = xx; high = xxx | | | | | | | | | | |

| | | | | | | | | | | |
|---|---|---|---|---|---|---|---|---|---|---|
| (mol [%])=(mol [%]) of components (b) or (c) in relation to the cationic groups in the polymer component (a). | | | | | | | | | | |

### Example 35

### Coating suspension preparation:

A coating composition was prepared mixing the formulation of example 1 with talc (50 % w/w ref. to polymer) and dispersing the powder compound in purified water by gentle stirring. The coating suspension had a content of dry solid of 18% w/w. Stirring is continued through the entire coating process.

### Coating process:

1800 g Quinidine sulphate tablets were loaded in a side vented coating pan Hi Coater LHC 30, Loedige) and coated with the coating suspension under appropriate conditions, i.e. a spray rate of approximately 7g / min coating suspension per kg cores and a bed temperature of approximately 30 - 35°C. Dry polymer weight gain was adjusted to 2 mg/cm² tablet surface. After coating the tablets were dried in the coater for 5 min at 45°C and for 2 hours on trays on an oven at 40°C.

### Results:

All tablets provided neutral taste over more than 10 minutes.

### Example 36

### Coating suspension preparation:

A coating composition was prepared mixing the formulation of example 1 with talc (50 % w/w ref. to polymer) and dispersing the powder compound in purified water by gentle stirring. The coating suspension had a content of dry solid of 18% w/w. Stirring is continued through the entire coating process.

### Coating process:

1800 g Quinidine sulphate tablets were loaded in a side vented coating pan Hi Coater LHC 30, Loedige) and coated with the coating suspension under appropriate conditions, i.e. a spray rate of approximately 7g / min coating suspension per kg cores and a bed temperature of approximately 30 - 35°C. Dry polymer weight gain was adjusted to 10 mg/cm² tablet surface. After coating the tablets were dried in the coater for 5 min at 45°C and for 2 hours on trays on an oven at 40°C.

### Results:

All tablets were tested for drug release in dissolution medium 1 and 2 and provided more than 90 % drug release in 15 min in both media. The same tablets tested in purified water provided a drug release of less than 5 % after 60 min.

### Example 37

### Coating suspension preparation:

A coating composition was prepared mixing the formulation of example 11 with talc (100 % w/w ref. to polymer) and dispersing the powder compound in purified water by gentle stirring. The coating suspension had a content of dry solid of 18% w/w. Stirring is continued through the entire coating process.

### Coating process:

1800 g Quinidine sulphate tablets were loaded in a side vented coating pan Hi Coater LHC 30, Loedige) and coated with the coating suspension under appropriate conditions, i.e. a spray rate of approximately 7g / min coating suspension per kg cores and a bed temperature of approximately 30 - 35°C. Dry polymer weight gain was adjusted to 2 mg/cm² tablet surface. After coating the tablets were dried in the coater for 5 min at 45°C and for 2 hours on trays on an oven at 40°C..

### Results:

All tablets provided an neutral taste over more than 10 minutes.

### Example 38

### Coating suspension preparation:

A coating composition was prepared mixing the formulation of example 11 with talc (100 % w/w ref. to polymer) and dispersing the powder compound in purified water by gentle stirring. The coating suspension had a content of dry solid of 18% w/w. Stirring is continued through the entire coating process.

### Coating process:

1800 g Quinidine sulphate tablets were loaded in a side vented coating pan Hi Coater LHC 30, Loedige) and coated with the coating suspension under appropriate conditions, i.e. a spray rate of approximately 7g / min coating suspension per kg cores and a bed temperature of approximately 30 - 35°C. Dry polymer weight gain was adjusted to 10 mg/cm² tablet surface. After coating the tablets were dried in the coater for 5 min at 45°C and for 2 hours on trays on an oven at 40°C.

### Results:

All tablets disintegrated in medium 1 in 2 - 5 min and in purified water in 30 - 60 min All tablets were tested in dissolution medium 1 and 2 and provided more than 90 % drug release in 15 min. The same tablets tested in purified water provided a drug release of less than 5 % after 60 min.

### Example 39

### Coating suspension preparation:

A coating composition was prepared mixing the formulation of example 1 with talc (50 % w/w ref. to polymer) and dispersing the powder compound in purified water by gentle stirring. The coating suspension had a content of dry solid of 18% w/w. Stirring is continued through the entire coating process.

### Coating process:

1800 g Silicagel tablets sulphate tablets were loaded in a side vented coating pan Hi Coater LHC 30, Loedige) and coated with the coating suspension under appropriate conditions, i.e. a spray rate of approximately 7g / min coating suspension per kg cores and a bed temperature of approximately 30 - 35°C. Dry polymer weight gain was adjusted to 10 mg/cm² tablet surface. After coating the tablets were dried in the coater for 5 min at 45°C and for 2 hours on trays on an oven at 40°C.

### Results:

Coated and uncoated tablets were stored in open containers at 40°C and 75 % rel. humidity. After 8 hours of testing the moistures uptake of the coated tablets was less than 15 % compared to the uncoated tablets set as 100 %.

### Example 40

### Coating suspension preparation:

A coating composition was prepared mixing the formulation of example 11 with talc (100 % w/w ref. to polymer) and dispersing the powder compound in purified water by gentle stirring. The coating suspension had a content of dry solid of 18% w/w. Stirring is continued through the entire coating process.

### Coating process:

1800 g Silicagel tablets sulphate tablets were loaded in a side vented coating pan Hi Coater LHC 30, Loedige) and coated with the coating suspension under appropriate conditions, i.e. a spray rate of approximately 7g / min coating suspension per kg cores and a bed temperature of approximately 30 - 35°C. Dray polymer weight gain was adjusted to 10 mg/cm² tablet surface. After coating the tablets were dried in the coater for 5 min at 50°C and for 2 hours on trays on an oven at 40°C..

### Results:

Coated and uncoated tablets were stored in open containers at 40°C and 75 % rel. humidity. After 8 hours of testing the moistures uptake of the coated tablets was less than 15 % compared to the uncoated tablets set as 100 %.

## Claims

1. A powdery or granulated composition comprising at least by 30 % by weight of a mixture of
(a) a copolymer composed of polymerized units of C₁- to C₄-alkyl esters of acrylic or methacrylic acid and of alkyl(meth)acrylate monomers with a tertiary amino group in the alkyl radical and
(b) 5 to 28 % by weight based on (a) of a salt of a fatty monocarboxylic acid having 10 to 18 carbon atoms, and
(c) 10 to 30 % by weight based on (a) of fatty monocarboxylic acid having 8 to 18 carbon atoms and/or a fatty alcohol having 8 to 18 carbon atoms.

2. Composition according to Claim 1 **characterised in that** the component (a) is a copolymer composed of polymerized units of 30 to 80% by weight of C₁- to C₄-alkyl esters of acrylic or of methacrylic acid and 70 to 20% by weight of alkyl(meth)acrylate monomers having a tertiary amino group in the alkyl radical.

3. Composition according to Claim 1 or 2, **characterized in that** the component (a) is a copolymer composed of polymerized units of 20 - 30% by weight of methyl methacrylate, 20 - 30% by weight of butyl methacrylate and 60 - 40% by weight of dimethylaminoethyl methacrylate.

4. Composition according to one or more Claims 1 to 3 **characterised in that** the component (b) is a salt of capric acid, lauric acid, myristic acid, palmitic acid or stearic acid or mixtures thereof.

5. Composition according to Claims 4 **characterised in that** the component (b) is sodium caprate.

6. Composition according to one or more Claims 1 to 5 **characterised in that** the component (c) is caprylic acid, capric acid, lauric acid, palmitic acid or stearic acid or mixtures thereof.

7. Composition according to one or more Claims 1 to 5 **characterised in that** the component (c) is capryl alcohol (1-octanol) or 1-dodecanol.

8. Composition according to one or more Claims 1 to 7 **characterised in that** up to 70 % by weight based on the total weight of the components (a), (b) and (c) of pharmaceutical, **nutraceutical** or cosmetical excipients which are different from the components (a), (b) and (c) are contained.

9. Composition according to Claim 8 **characterised in that** the excipients are selected from the classes of antioxidants, brighteners, flavouring agents, flow aids, fragrances, glidants, penetration-promoting agents, pigments, plasticizers, polymers, pore-forming agents or stabilizers.

10. Composition according to one or more Claims 1 to 9 **characterised in that** the composition is present in a dissolved form of an aqueous dispersion with a dry weight content of 5 to 40 % (weight/volume).

11. Composition according to one or more Claims 1 to 10 **characterized by** a dispersion or solution preparation time of less than 3 hours measured from stirring the dry powdery or granulate mixture into water at room temperature, further stirring and thereby dissolving the components until a clear solution or a dispersion respectively has be been produced.

12. Process for preparing a composition according to one ore more Claims 1 to 11, characterized that the components (a), (b) and (c) are intermixed with each other by powder mixture, dry granulation, wet granulation, melt granulation, spray drying or freeze drying.

13. Process according to Claim 12, **characterized in that** the components (a), (b) and (c) are intermixed with each other by wet granulation whereby the polymer component (a) is used in the form an organic solution.

14. Use of a composition according to one or more Claims 1 to 11 as a coating or binding agent for pharmaceutical, nutraceutical or cosmetical compositions.

## Patentansprüche

1. Pulver- oder granulatförmige Zusammensetzung, umfassend mindestens 30 Gew.-% einer Mischung von
(a) einem Copolymer, das aus polymerisierten Einheiten von C₁- bis C₄-Alkylestern der Acryl- oder Methacrylsäure und Alkyl(meth)-acrylat-Monomeren mit einer tertiären Aminogruppe im Alkylrest besteht, und
(b) 5 bis 28 Gew.-%, bezogen auf (a), eines Salzes einer Fettmonocarbonsäure mit 10 bis 18 Kohlenstoffatomen und
(c) 10 bis 30 Gew.-%, bezogen auf (a), einer Fettmonocarbonsäure mit 8 bis 18 Kohlenstoffatomen und/oder eines Fettalkohols mit 8 bis 18 Kohlenstoffatomen.

2. Zusammensetzung nach Anspruch 1, **dadurch gekennzeichnet, dass** es sich bei der Komponente (a) um ein Copolymer, das aus polymerisierten Einheiten von 30 bis 80 Gew.-% C₁- bis C₄-Alkylestern der Acryl- oder Methacrylsäure und 70 bis 20 Gew.-% Alkyl(meth)acrylat-Monomeren mit einer tertiären Aminogruppe im Alkylrest besteht, handelt.

3. Zusammensetzung nach Anspruch 1 oder 2, **dadurch gekennzeichnet, dass** es sich bei der Komponente (a) um ein Copolymer, das aus polymerisierten Einheiten von 20-30 Gew.-% Methylmethacrylat, 20-30 Gew.-% Butylmethacrylat und 60-40 Gew.-% Dimethylaminoethylmethacrylat besteht, handelt.

4. Zusammensetzung nach einem oder mehreren der Ansprüche 1 bis 3, **dadurch gekennzeichnet, dass** es sich bei der Komponente (b) um ein Salz von Caprinsäure, Laurinsäure, Myristinsäure, Palmitinsäure, Stearinsäure oder Mischungen davon handelt.

5. Zusammensetzung nach Anspruch 4, **dadurch gekennzeichnet, dass** es sich bei der Komponente (b) um Natriumcaprat handelt.

6. Zusammensetzung nach einem oder mehreren der Ansprüche 1 bis 5, **dadurch gekennzeichnet, dass** es sich bei der Komponente (c) um Caprylsäure, Caprinsäure, Laurinsäure, Palmitinsäure oder Stearinsäure oder Mischungen davon handelt.

7. Zusammensetzung nach einem oder mehreren der Ansprüche 1 bis 5, **dadurch gekennzeichnet, dass** es sich bei der Komponente (c) um Caprylalkohol (1-Octanol) oder 1-Dodecanol handelt.

8. Zusammensetzung nach einem oder mehreren der Ansprüche 1 bis 7, **dadurch gekennzeichnet, dass** bis zu 70 Gew.-%, bezogen auf das Gesamtgewicht der Komponenten (a), (b) und (c), pharmazeutischer, nutrazeutischer oder kosmetischer Hilfsstoffe, die von den Komponenten (a), (b) und (c) verschieden sind, enthalten sind.

9. Zusammensetzung nach Anspruch 8, **dadurch gekennzeichnet, dass** die Hilfsstoffe aus den Klassen Antioxidantien, Aufheller, Geschmacksmittel, Rieselhilfen, Duftstoffe, Gleitmittel, Penetrationsförderer, Pigmente, Weichmacher, Polymere, Porenbildner oder Stabilisatoren ausgewählt sind.

10. Zusammensetzung nach einem oder mehreren der Ansprüche 1 bis 9, **dadurch gekennzeichnet, dass** die Zusammensetzung in einer gelösten Form einer wässrigen Dispersion mit einem Trockengewichtsgehalt von 5 bis 40% (Gewicht/Volumen) vorliegt.

11. Zusammensetzung nach einem oder mehreren der Ansprüche 1 bis 10, **gekennzeichnet durch** eine Dispersions- bzw. Lösungsherstellungszeit von weniger als 3 Stunden, gemessen ab dem Einrühren der trockenen pulver- oder granulatförmigen Mischung in Wasser bei Raumtemperatur, Weiterrühren und **dadurch** Lösen der Komponenten bis zum Erhalt einer klaren Lösung bzw. Dispersion.

12. Verfahren zur Herstellung einer Zusammensetzung nach einem oder mehreren der Ansprüche 1 bis 11, **dadurch gekennzeichnet, dass** man die Komponenten (a), (b) und (c) durch Pulvermischen, Trockengranulation, Feuchtgranulation, Schmelzegranulation, Sprühtrocknen oder Gefriertrocknen miteinander vermischt.

13. Verfahren nach Anspruch 12, **dadurch gekennzeichnet, dass** man die Komponenten (a), (b) und (c) durch Feuchtgranulation miteinander vermischt, wobei die Polymerkomponente (a) in Form einer organischen Lösung verwendet wird.

14. Verwendung einer Zusammensetzung nach einem oder mehreren der Ansprüche 1 bis 11 als Überzugs- oder Bindemittel für pharmazeutische, nutrazeutische oder kosmetische Zusammensetzungen.

## Revendications

1. Composition pulvérulente ou granulée comprenant au moins environ 30 % en poids d'un mélange de
(a) un copolymère composé de motifs polymérisés constitués d'esters alkyliques en C₁ à C₄ de l'acide acrylique ou méthacrylique et de monomères (méth)acrylates d'alkyle renfermant un groupe amino tertiaire dans le radical alkyle et
(b) 5 à 28 % en poids sur la base de (a) d'un sel d'un acide monocarboxylique gras ayant 10 à 18 atomes de carbone et
(c) 10 à 30 % en poids sur la base de (a) d'acide monocarboxylique gras ayant 8 à 18 atomes de carbone et/ou d'un alcool gras ayant 8 à 18 atomes de carbone.

2. Composition selon la revendication 1 **caractérisée en ce que** le composant (a) est un copolymère composé de motifs polymérisés constitués de 30 à 80 % en poids d'esters alkyliques en C₁ à C₄ de l'acide acrylique ou de l'acide méthacrylique et de 70 à 20 % en poids de monomères (méth)acrylates d'alkyle ayant un groupe amino tertiaire dans le radical alkyle.

3. Composition selon la revendication 1 ou 2, **caractérisée en ce que** le composant (a) est un copolymère composé de motifs polymérisés constitués de 20-30 % en poids de méthacrylate de méthyle, 20-30 % en poids de méthacrylate de butyle et 60-40 % en poids de méthacrylate de diméthylaminoéthyle.

4. Composition selon une ou plusieurs des revendications 1 à 3 **caractérisée en ce que** le composant (b) est un sel de l'acide caprique, de l'acide laurique, de l'acide myristique, de l'acide palmitique ou de l'acide stéarique ou de mélanges de ceux-ci.

5. Composition selon la revendication 4 **caractérisée en ce que** le composant (b) est le caprate de sodium.

6. Composition selon une ou plusieurs des revendications 1 à 5 **caractérisée en ce que** le composant (c) est l'acide caprylique, l'acide caprique, l'acide laurique, l'acide palmitique ou l'acide stéarique ou des mélanges de ceux-ci.

7. Composition selon une ou plusieurs des revendications 1 à 5 **caractérisée en ce que** le composant (c) est l'alcool caprylique (le 1-octanol) ou le 1-dodécanol.

8. Composition selon une ou plusieurs des revendications 1 à 7 **caractérisée en ce que** jusqu'à 70 % en poids sur la base du poids total des composants ( a ), ( b ) e t ( c ) d'excipients pharmaceutiques, nutraceutiques ou cosmétiques qui sont différents des composants (a), (b) et (c) sont contenus.

9. Composition selon la revendication 8 **caractérisée en ce que** les excipients sont choisis parmi les classes des antioxydants, des azurants optiques, des agents aromatisants, des adjuvants d'écoulement, des parfums, des agents de glissement, des agents favorisant la pénétration, des pigments, des plastifiants, des polymères, des agents porogènes ou des stabilisants.

10. Composition selon une ou plusieurs des revendications 1 à 9 **caractérisée en ce que** la composition est présente sous une forme dissoute d'une dispersion aqueuse ayant une teneur en matières sèches en poids de 5 à 40 % (en poids/volume).

11. Composition selon une ou plusieurs des revendications 1 à 10 **caractérisée par** un temps de préparation de dispersion ou de solution inférieur à 3 heures mesuré à partir de l'agitation du mélange sec pulvérulent ou granulé dans de l'eau à température ambiante, pendant l'agitation encore et de cette manière la dissolution des composants et jusqu'à ce qu'une solution transparente ou une dispersion respectivement ait été produite.

12. Procédé pour la préparation d'une composition selon une ou plusieurs des revendications 1 à 11, **caractérisé en ce que** les composants (a), (b) et (c) sont mélangés les uns avec les autres par mélange de poudres, granulation à sec, granulation en voie humide, granulation à l'état fondu, séchage par pulvérisation ou lyophilisation.

13. Procédé selon la revendication 12, **caractérisé en ce que** les composants (a), (b) et (c) sont mélangés les uns avec les autres par granulation en voie humide, le composant polymère (a) étant utilisé sous la forme d'une solution organique.

14. Utilisation d'une composition selon une ou plusieurs des revendications 1 à 11 comme enrobage ou agent liant pour des compositions pharmaceutiques, nutraceutiques ou cosmétiques.
